(19) Europäisches Patentamt
European Patent Office
Office européen des brevets

(11) Veröffentlichungsnummer: **0 167 996**
**A1**

(12) # EUROPÄISCHE PATENTANMELDUNG

(21) Anmeldenummer: 85108320.4

(22) Anmeldetag: 05.07.85

(51) Int. Cl.4: C 07 C 85/06
C 07 C 85/24, C 07 C 87/50
C 07 C 87/36

(30) Priorität: 12.07.84 DE 3425629

(43) Veröffentlichungstag der Anmeldung:
15.01.86 Patentblatt 86/3

(84) Benannte Vertragsstaaten:
AT BE CH DE FR GB IT LI NL

(71) Anmelder: BASF Aktiengesellschaft
Carl-Bosch-Strasse 38
D-6700 Ludwigshafen(DE)

(72) Erfinder: Goetz, Norbert, Dr.
Schoefferstrasse 25
D-6520 Worms 1(DE)

(72) Erfinder: Hupfer, Leopold, Dr.
Waltershoehe 3
D-6701 Friedelsheim(DE)

(72) Erfinder: Franzischka, Wolfgang, Dr.
Franz-Lind-Strasse 1
D-6713 Freinsheim(DE)

(72) Erfinder: Reiss, Wolfgang, Dr.
Bannwasserstrasse 70
D-6700 Ludwigshafen(DE)

(72) Erfinder: Koernig, Wolfgang, Dr.
Dachsweg 5
D-6901 Dossenheim(DE)

(54) **Verfahren zur Herstellung von cyclischen, am Ring substituierten primären Aminen.**

(57) Aromatische Amine (substituierte Aniline) der allgemeinen Formel $R^1$-$NH_2$ und/oder entsprechende cycloaliphatische Amine der allgemeinen Formel $R^2$-$NH_2$, wobei $R^1$ einen durch aliphatische Gruppen mit 1 bis 20 Kohlenstoffatomen und cycloaliphatischen Gruppen mit 5 bis 20 Kohlenstoffatomen substituierten Phenylring und $R^2$ einen entsprechend substituierten Cyclohexylring bedeutet, erhält man aus entsprechenden Phenolen in Gegenwart eines ggf. zurückgeführten cycloaliphatischen Amins $R^2$-$NH_2$ mit überschüssigem Ammoniak und überschüssigem Wasserstoff an einem Hydrier-/Dehydrierkatalysator, wobei die Umsetzung bei im wesentlichen atmosphärischem Druck in der Gasphase fortlaufend in wenigstens zwei hintereinandergeschalteten Reaktionszonen derart vorgenommen wird, daß in einer ersten Reaktionszone eine niedrigere Temperatur und in einer zweiten Reaktionszone eine höhere Temperatur eingehalten wird und/oder in den Reaktionszonen unterschiedlich reaktive Katalysatoren angeordnet sind. Die Katalysatoren können z.B. ein Palladium-, Ruthenium-, Rhodium- oder Platinkatalysator bzw. ein Palladium-, Platin-Kupfer- oder Silberkatalysator sein und sind Trägerkatalysatoren mit Aluminiumoxid als Träger, der zusätzlich einen basischen Stoff und/oder Zink, Chrom, Mangan, Eisen, Kobalt oder Nickel enthalten kann.

EP 0 167 996 A1

Croydon Printing Company Ltd

Verfahren zur Herstellung von cyclischen, am Ring substituierten primären Aminen

Die einstufige Überführung von Phenolen mit Ammoniak und Wasserstoff in Cyclohexylamine in Gegenwart von Edelmetallkatalysatoren ist bereits mehrfach beschrieben worden (JA-OS 4 034 677, FR-PS 1 427 543, GB-PS 1 031 169, DE-PS 12 76 032, EP-A 22 751). Dabei wird fast ausschließlich die Bildung der gesättigten cyclischen Amine beobachtet, während aromatische Amine kaum entstehen.

Ein Verfahren zur direkten Herstellung von aromatischen Aminen aus Phenolen ist ebenfalls schon bekannt (DE-OS 2 208 827, US-PSen 3 931 298, 3 960 962). Bei diesem Verfahren findet die Umsetzung diskontinuierlich (absatzweise) in der Flüssigphase in Gegenwart eines Edelmetallkatalysators und einer Cyclohexanonverbindung statt, die dem Reaktionsgemisch als Katalysator anfangs in einer gewissen Menge zugesetzt oder aus dem Phenol durch Zugabe einer begrenzten Menge Wasserstoff oder eines Wasserstoffspenders erzeugt wird. Bei einem aus der EP-PS 31 859 bekannten Verfahren wird als der Wasserstoffspender das entsprechend substituierte, gesättigte cyclische Amin verwendet. Es handelt sich bei diesem Verfahren offensichtlich um eine sog. Eintopfsynthese mit einer unmittelbaren Aneinanderreihung verschiedener Verfahrensschritte, wie Überführung des Phenols in das Cyclohexanon, das dann mit vorhandenem Ammoniak zum cyclischen Imin reagiert, welches wiederum in Abhängigkeit von der vorhandenen Wasserstoffmenge entweder zum gesättigten cyclischen Amin oder zum aromatischen Amin umgesetzt wird (Journal of Catalysis 72, S. 121 - 128 (1981)). Nachteilig bei diesem Verfahren ist der Umstand, daß als Reaktionsprodukte immer Phenol/Anilin-Gemische mit engem Siedepunktsabstand entstehen, die nur mit erheblichem Aufwand (Destillation; hohe Energiekosten) zu trennen sind. Weiterhin können bedingt durch die diskontinuierliche Arbeitsweise in einer definierten Zeiteinheit nur vergleichsweise geringe Mengen an Wertprodukt in einem vorgegebenen Reaktorvolumen hergestellt werden, d.h. dieses Verfahren liefert schlechte Raum-Zeit-Ausbeuten.

Die - ähnlich verlaufende - Umsetzung von Phenol/Cyclohexanol-Mischungen mit Ammoniak in der Flüssigphase, wobei Cyclohexanol als Wasserstoffspender benutzt wird und als Reaktionsprodukt Anilin/Cyclohexylamin-Mischungen erhalten werden (Chem. Lett. 1980, 3, S. 239 - 240), hat den Nachteil, daß, bedingt durch die erhöhten Temperaturen und die Durchführung in der Flüssigphase (hohes Eigenvolumen des vorhandenen Ammoniaks) schlechte Raum-Zeit-Ausbeuten erhalten werden. Außerdem muß der Wasserstoffspender Cyclohexanol in einem separaten Schritt hergestellt werden.

Es wurde nun gefunden, daß man substituierte Aniline, die am Ring mindestens einen Alkyl- oder Cycloalkyl-Substituenten tragen, mit wechselnden Mengen an entsprechenden Cyclohexylaminen erhält, wenn man eine Mischung aus einem substituierten Phenol und dem entsprechenden Cyclohexylamin mit überschüssigem Ammoniak und überschüssigem Wasserstoff an einem Hydrier-/ Dehydrier-Katalysator umsetzt, wobei die Reaktion fortlaufend in der Gasphase bei im wesentlichen atmosphärischem Druck so durchgeführt wird, daß das Reaktionsgemisch mindestens zwei unterschiedliche Reaktionszonen entweder zunächst bei tieferer Temperatur und anschließend bei höherer Temperatur oder an unterschiedlich reaktiven Katalysatoren durchläuft. Als Katalysatoren werden für die erste Reaktionszone (am Eingang des Reaktors) bevorzugt Palladium, Ruthenium, Rhodium, Platin verwendet und z.B. eine Temperatur von 100 bis 250°C eingehalten. Für die zweite Reaktionszone wird bevorzugt Palladium, Platin, Kupfer und Silber verwendet und es wird z.B. eine Temperatur von 180 bis 300°C eingehalten. Als Katalysatoren werden Trägerkatalysatoren benutzt, die bevorzugt aluminiumoxidhaltige oder aus Aluminiumoxid aufgebaute Träger besitzen und zusätzlich noch einen basischen Stoff und/oder Zink Chrom, Mangan, Eisen, Kobalt oder Nickel enthalten können. Technisch ließe sich auch Anilin selbst nach diesem Verfahren aus Phenol gewinnen, was sich aber aus anderen Gründen nicht lohnt. So wird das Verfahren zur Herstellung substituierter Aniline und ggf. Cyclohexylamine verwendet. Als Ringsubstituenten kommen aliphatische Gruppen mit 1 bis 20 Kohlenstoffatomen und cycloaliphatische Gruppen mit 5 bis 20 Kohlenstoffatomen in Frage.

Das erfindungsgemäße Verfahren hat gegenüber den bekannten Verfahren Vorteile:
Die eingesetzten Phenol-/Cyclohexylamin-Mischungen sind bei Raumtemperatur flüssig und ermöglichen eine einfache Technologie für die Zufuhr zum Reaktor, anders als die zumeist kristallinen reinen Phenole. Die Kopplung eines stark exothermen Prozesses (hydrierende Aminierung des Phenols) mit einem endothermen Prozeß (Dehydrierung des Cyclohexylamins) verläuft mit ausgeglichener Energiebilanz, so daß nur beim Anfahren Energie zugeführt werden muß; die eigentliche Umsetzung läuft weitgehend ohne Energiezufuhr ab. Die in der ersten Reaktionszone angefallene Energie wird mit der zugeführten Gasmischung und dem Reaktionsprodukt als Wärmeträger in die zweite Reaktionszone transportiert, wo sie für den Ablauf der endothermen Dehydrierung benötigt wird. Da als Reaktionsendprodukte Mischungen aus leicht trennbaren substituierten Cyclohexylaminen und aromatischen Aminen anfallen, die frei von den als Ausgangsprodukten benutzten substituierten Phenolen und weiteren Verunreinigungen sind, werden hier nochmals Energiekosten eingespart.

Über die Reaktionsbedingungen (Reaktionstemperatur; Zusammensetzung der Gasmischung) kann man sehr leicht steuern, ob in der Mischung der Reaktionsprodukte das cycloaliphatische oder das aromatische Amin überwiegt. So werden bei relativ niedriger Reaktionstemperatur (besonders in der zweiten Reaktionszone) und hohem Wasserstoffüberschuß fast ausschließlich substituierte Cyclohexylamine und bei hoher Reaktionstemperatur (speziell in der zweiten Reaktionszone) und hohem Ammoniaküberschuß praktisch nur aromatische Amine gewonnen. Vorteilhaft nimmt man aber als Einsatzstoff eine Mischung aus Phenol und cycloaliphatischem Amin im Molverhältnis von etwa 1:1 und setzt so um, daß in der erhaltenen Endprodukte-Mischung cycloaliphatisches Amin und aromatisches Amin ebenfalls etwa im Molverhältnis 1:1 vorliegen. Dabei wird sowohl eine ausgeglichene Energie-, wie auch Wasserstoffbilanz erhalten und gleichzeitig genügend cycloaliphatisches Amin erzeugt, das zur Lösung der kristallinen Phenole zurückgeführt werden kann. Natürlich läßt sich das Verfahren so steuern, daß auch bei einem anderen Mengenverhältnis von Phenol zu Amin jeweils das gesamte cycloaliphatische Amin zurückgeführt werden kann.

Für den Fall der Herstellung des 2,6-Dimethylanilins aus 2,6-Dimethylphenol läuft die Reaktion nach folgendem Schema ab:

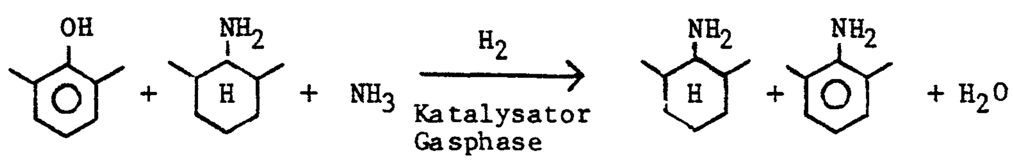


Die Bildung von normalerweise typischen Nebenprodukten, wie Cyclohexanolen, Dicyclohexylaminen, Diphenylaminen u. a. kann bei diesem Verfahren vollständig unterdrückt werden.

Als substituierte Phenole können für das erfindungsgemäße Verfahren eingesetzt werden beispielsweise o-, m- und p-Kresol, o-Ethylphenol, o-n-Butylphenol, o-sek.-Butylphenol, 2,4-Dimethylphenol, 2,6-Dimethylphenol, 2,3,6-Trimethylphenol, 2,4,6-Trimethylphenol, 2-Cyclohexylphenol, 2,6-Dimethyl-3-cyclohexyl-phenol, 2,6-Diethylphenol, 2,5-Diisopropylphenol, 2-Methyl-6-sek.-butylphenol, 3-tert.-Butylphenol, 2,6-Diisopropylphenol, 2,6-Di-sek.-butylphenol, 2,6-Dicyclohexyl-phenol.

Ammoniak und Wasserstoff werden - bezogen auf das eingesetzte substituierte Phenol - in einem z.B. 2- bis 40molaren Überschuß angewendet. Untereinander können sie z.B. im Molverhältnis von 10:1 bis 1:10 vorliegen.

Als technische Verwirklichung des Begriffes "Reaktionszone" werden vor allem Rohrreaktoren verwendet; im Prinzip kommt es aber nur darauf an,

eine Rückvermischung vom Ausgang der Anlage, d.h. dem Ende der letzten Reaktionszone zum Eingang, d.h. dem Beginn der ersten Reaktionszone zu vermeiden, was sich in Rohrreaktoren und der sich darin ausbildenden Pfropfenströmung am leichtesten erreichen läßt. Der Begriff "Rohrreaktor", wie er hier verwendet wird, ist also im weitesten Sinne gemeint.

Der Katalysator kann jeweils in einem Festbett oder auch als Wirbelbett angeordnet sein. Als Katalysatoren kommen Hydrier-/Dehydrier-Katalysatoren in Frage, die zweckmäßig "maßgeschneidert" für die erste Reaktionszone, in der überwiegend die hydrierende Aminierung abläuft, und für die zweite Reaktionszone, in der sich weitgehend die Dehydrierung abspielt, eingesetzt werden. In der ersten Reaktionszone mit einem bevorzugten Temperaturbereich von 100 bis 250°C werden die besten Ergebnisse mit Ruthenium-, Rhodium-, Palladium- und/oder Platin-Katalysatoren erhalten. In der zweiten Reaktionszone (bevorzugter Temperaturbereich: 180 - 300°C) bewährten sich Palladium-, Platin-, Kupfer- und/oder Silberkatalysatoren. Die aktiven Bestandteile des Katalysators (Edelmetalle) befinden sich auf einem vorzugsweise im wesentlichen aus Aluminiumoxid aufgebauten Träger. Bevorzugt enthält der Träger noch Zusätze, beispielsweise basische Komponenten, wie Oxide, Hydroxide oder Carbonate der Alkalimetalle, vorzugsweise des Lithiums und Natriums, der Erdalkalimetalle, vorzugsweise des Magnesiums und Calciums, der Seltenen Erdmetalle, vorzugsweise des Praseodyms oder Oxiden von Schwermetallen, wie Zink, Chrom, Mangan, Eisen, Kobalt oder Nickel. Die Zusätze können im Gemisch mit dem Aluminiumoxid vorliegen oder durch gemeinsames Tempern in Spinelle überführt werden. Eine Beschreibung geeigneter Katalysatoren findet sich beispielsweise in der DE-OS 32 09 148 oder der EP 53 817.

Die Katalysatoren werden entweder durch gemeinsames Kneten der Zusätze (in Form der Metalloxide) mit Aluminiumoxid, thermische Nachbehandlung (Tempern) bei 400 bis 900°C Tränken mit einer das Edelmetall enthaltenden Lösung oder durch Tränken des Trägers mit einer Lösung der Zusätze und des Hydriermetalls, z.B. in Form von Lösungen ihrer Nitrate, Chloride, Formiate, Oxalate oder Ammoniakate und darauf folgendes Tempern bei 400 bis 900°C hergestellt. Soll Spinellbildung erzielt werden, muß nach dem Kneten bzw. Tränken des Aluminiumoxids mit dem Oxid bzw. der Lösung der Zusatzkomponente eine Temperatur von 900 bis 1300°C erreicht werden (siehe Ullmanns Encyklopädie der technischen Chemie, 3. Auflage (1955) Band 6, S. 242 - 244, Gmelin, System-Nr. 35, Al Tl 1934 - 1935, S. 26 - 28).

Der Edelmetallgehalt des Katalysators, bezogen auf das Trägermaterial, liegt gewöhnlich zwischen 0,05 bis 15 Gew.-%. Das Gewichtsverhältnis der

Zusätze zum Edelmetall kann sehr unterschiedlich sein. In der Regel liegt es zwischen 1 000 : 1 bis 1 : 50. Man verwendet den Katalysator beispielsweise in Form von Strängen oder als Pulver, je nach vorgesehenem Verwendungszweck.

Die nach dem Verfahren der Erfindung hergestellten Verbindungen werden z.B. zur Herstellung von Wirkstoffen in Pflanzenschutzmitteln verwendet (DE-AS 2 305 495, DE-OS 2 648 008, DE-OS 2 513 732, DE-OS 2 515 091).

Beispiel 1

In ein 1,2 l fassendes Reaktionsrohr von 500 mm Länge, welches zwei getrennt heizbare Zonen aufweist, wird ein Strangkatalysator (3 mm Durchmesser, 10 mm Länge) eingefüllt, der 0,5 Gew.-% Palladium auf einem Träger aus 19,4 Gew.-% Magnesiumoxid und 80,6 Gew.-% Aluminiumoxid enthält. Nun wird in der ersten Reaktionszone (Ofeneingang) eine Temperatur von 193°C und in der zweiten Reaktionszone eine Temperatur von 205°C eingestellt. Über dieses Katalysatorbett wird aus seinem Verdampfer stündlich eine auf 193°C vorerhitzte Mischung aus 61 g 2,6-Dimethylphenol und 63 g 2,6-Dimethylcyclohexylamin (Isomerengemisch) geleitet. Gleichzeitig wird eine ebenfalls auf 193°C vorerhitzte Mischung aus 300 Nl Wasserstoff und 100 Nl Ammoniak pro Stunde durch den Reaktor im Gleichstrom geschickt. Sobald konstante Bedingungen erreicht sind, kann der Reaktor - gute Wärmeisolierung vorausgesetzt - weitgehend ohne zusätzliche Heizung betrieben werden. Das Reaktionsprodukt wird - sobald es den Reaktor verläßt - abgekühlt. Es besteht nach gaschromatographischer Analyse aus 52 Gew.-% 2,6-Dimethylanilin ($K_p$ = 216°C) und 48 Gew.-% 2,6--Dimethylcyclohexylamin ($K_p$ = 167 - 168°C). Beträgt die Temperatur in der 1. Reaktionszone 208°C und in der zweiten Reaktionszone 222°C - unter sonst gleichen Bedingungen - so erhält man ein Reaktionsprodukt aus 84 Gew.-% 2,6-Dimethylanilin und 16 Gew.-% 2,6-Dimethylcyclohexylamin. Die beiden Amine können durch Destillation leicht voneinander getrennt werden; das zurückgewonnene 2,6-Dimethylcyclohexylamin dient zum Lösen des kristallinen 2,6-Dimethylphenols und wird der Reaktionszone wieder zugeführt. Bei vollständiger Überführung des eingesetzten 2,6-Dimethylphenols in 2,6-Dimethylanilin erhält man aus je 100 g 2,6-Dimethylphenol 97,5 g 2,6-Dimethylanilin, entsprechend einer Ausbeute von 98,3 % d. Th.

Beispiel 2

Man verfährt entsprechend Beispiel 1, hat aber in der ersten Reaktionszone einen Katalysator mit 1,0 Gew.-% Ruthenium auf einem Zink-Aluminium-Spinell-Träger und in der zweiten Reaktionszone einen Katalysator mit 1,0 Gew.-% Palladium auf einem Lithium-Aluminium-Spinell-Träger. Werden beide Reaktionszonen auf einer Temperatur von 185°C gehalten, so erhält man ein Umsetzungsprodukt mit folgender Zusammensetzung: 66,0 Gew.-%, 2,6-Dimethylanilin und 34,0 Gew.-% 2,6-Dimethylcyclohexylamin.

Wird eine Mischung aus 75 g 2,6-Dimethylphenol und 25 g 2,6-Dimethylcyclohexylamin pro Stunde zugeführt, bleibt die Zusammensetzung des Endproduktes unverändert. Wird die stündlich zugeführte Wasserstoffmenge von 300 Nl auf 400 Nl erhöht, so besteht das unter diesen Bedingungen gebildete Reaktionsprodukt aus 58,0 Gew.-% 2,6-Dimethylanilin und 42,0 Gew.-% 2,6-Dimethylcyclohexylamin.

Beispiel 3

Man verfährt entsprechend Beispiel 1, führt aber stündlich eine Mischung aus 102 g 2,6-Dimethyl-3-cyclohexyl-phenol und 105 g 2,6-Dimethyl-3-cyclohexyl-cyclohexylamin zunächst über einen Katalysator, der 0,5 Gew.-% Rhodium auf einem Kobalt-Aluminium-Spinell-Träger enthält und auf 198°C erhitzt ist, anschließend über einen Katalysator, der 0,5 Gew.-% Platin und 5,0 Gew.-% Praseodymoxid auf Aluminiumoxid enthält und auf 203°C erhitzt ist. Man erhält 72 Gew.-% 2,6-Dimethyl-3-cyclohexyl-anilin ($K_p$ = 115 - 116°C/0,2 mbar) und 28 Gew.-% 2,6-Dimethyl-3-cyclohexyl-cyclohexylamin ($K_p$ = 95 - 98°C/ 0,2 mbar).

Beispiel 4

Man verfährt entsprechend Beispiel 1, führt aber stündlich eine Mischung aus 88 g 2,6-Diisopropylphenol und 92 g 2,6-Diisopropylcyclohexylamin zunächst über einen Katalysator, der 1,0 Gew.-% Ruthenium auf einem Magnesium-Aluminium-Spinell-Träger enthält und auf 217°C erhitzt ist, anschließend über einen Katalysator, der 30 Gew.-% Kupfer auf einem Träger aus 22 Gew.-% Zinkoxid, 3 Gew.% Chromoxid und 75 Gew.-% Aluminiumoxid enthält und auf 232°C erhitzt ist. Das Reaktionsprodukt besteht aus 76 Gew.-% 2,6-Diisopropylanilin ($K_p$ = 257°C) und 24 Gew.-% 2,6-Diisoproypylcyclohexylamin ($K_p$ = 239 - 240°C).

Beispiel 5

Man verfährt entsprechend Beispiel 1, führt aber stündlich eine Mischung aus 75 g 3-tert.-Butylphenol und 78 g 3-tert.-Butylcyclohexylamin zunächst über einen Katalysator, der 1,5 Gew.-% Palladium auf einem Nickel-Aluminium-Spinell-Träger enthält und auf 190°C erhitzt ist, anschließend über einen Katalysator, der 0,2 Gew.-% Platin auf einem Träger aus 19,4 Gew.-% Calciumoxid und 80,6 Gew.-% Aluminiumoxid enthält und auf 206°C erhitzt ist. Man gewinnt eine Mischung aus 78 Gew.-% 3-tert.-Butylanilin ($K_p$ = 123 - 124°C/27 mbar) und 22 Gew.-% 3-tert.-Butylcyclohexylamin ($K_p$ = 84 - 85°C/27 mbar).

## Patentansprüche

1. Verfahren zur Herstellung von aromatischen Aminen der allgemeinen Formel $R^1-NH_2$ und/oder cycloaliphatischen Aminen der allgemeinen Formel $R^2-NH_2$, wobei $R^1$ einen durch aliphatische Gruppen mit 1 bis 20 Kohlenstoffatomen und cycloaliphatischen Gruppen mit 5 bis 20 Kohlenstoffatomen substituierten Phenylring und $R^2$ einen entsprechend substituierten Cyclohexylring bedeutet, durch Umsetzung eines entsprechenden Phenols der allgemeinen Formel $R^1-OH$ in Gegenwart eines ggf. zurückgeführten cycloaliphatischen Amins $R^2-NH_2$, mit überschüssigem Ammoniak und überschüssigem Wasserstoff an einem Hydrier-/Dehydrierkatalysator, dadurch gekennzeichnet, daß die Umsetzung bei im wesentlichen atmosphärischem Druck in der Gasphase fortlaufend in wenigstens zwei hintereinandergeschalteten Reaktionszonen vorgenommen wird, wobei in einer ersten Reaktionszone eine niedrigere Temperatur und in einer zweiten Reaktionszone eine höhere Temperatur eingehalten wird und/oder in den Reaktionszonen unterschiedlich reaktive Katalysatoren angeordnet sind.

2. Verfahren nach Anspruch 1, dadurch gekennzeichnet, daß in einer ersten Reaktionszone ein Palladium-, Ruthenium-, Rhodium- oder Platinkatalysator und in einer zweiten Reaktionszone ein Palladium-, Platin-, Kupfer- oder Silberkatalysator angeordnet ist.

3. Verfahren nach Anspruch 1 oder 2, dadurch gekennzeichnet, daß der Katalysator jeweils ein Trägerkatalysator ist und der Träger Aluminiumoxid enthält oder aus Aluminiumoxid aufgebaut ist.

4. Verfahren nach Anspruch 3, dadurch gekennzeichnet, daß der Träger zusätzlich einen basischen Stoff und/oder Zink, Chrom, Mangan, Eisen, Kobalt oder Nickel enthält.

5. Verfahren nach einem der Ansprüche 1 bis 4, dadurch gekennzeichnet, daß in einer ersten Reaktionszone eine Temperatur von 100 bis 250°C und in einer zweiten Reaktionszone eine Temperatur von 180 bis 300°C eingehalten wird.

0167996

Europäisches Patentamt

# EUROPÄISCHER RECHERCHENBERICHT

Nummer der Anmeldung

EP 85 10 8320

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,Y | EP-A-0 053 696 (BASF)<br>* Ansprüche * | 1 | C 07 C 85/06<br>C 07 C 85/24<br>C 07 C 87/50<br>C 07 C 87/36 |
| | --- | | |
| D,Y | EP-A-0 053 819 (BASF)<br>* Ansprüche * | 1 | |
| | --- | | |
| D,Y | EP-A-0 053 817 (BASF)<br>* Ansprüche * | 1 | |
| | --- | | |
| D,Y | EP-A-0 022 751 (CIBA-GEIGY)<br>* Ansprüche * | 1 | |
| | --- | | |
| Y | EP-A-0 108 323 (BASF)<br>* Ansprüche * | 1 | |
| | --- | | |
| D,Y | US-A-3 931 298 (J.C. WOLLENSAK)<br>* Ansprüche * | 1 | RECHERCHIERTE SACHGEBIETE (Int Cl 4) |
| | ----- | | C 07 C 85/00<br>C 07 C 87/00 |

Der vorliegende Recherchenbericht wurde für alle Patentansprüche erstellt

| Recherchenort | Abschlußdatum der Recherche | Prüfer |
|---|---|---|
| DEN HAAG | 08-10-1985 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument